# EUROPÄISCHE PATENTSCHRIFT

(11) **EP 1 056 769 B1**
(45) Veröffentlichungstag und Bekanntmachung des Hinweises auf die Patenterteilung: **19.06.2002**
(21) Anmeldenummer: 98965592.3
(22) Anmeldetag: 30.11.1998
(51) Int. Cl.: C07J 53/00, A61K 31/565

(54) **15-METHYL-CYCLOPROPANOSTEROIDE UND VERFHAREN ZU IHRER HERSTELLUNG**
15-METHYL-CYCLOPROPANOSTEROIDS AND METHOD FOR THE PRODUCTION THEREOF
15-METHYL-CYCLOPROPANOSTEROIDES ET LEUR PROCEDE DE FABRICATION

(30) Priorität: 02.12.1997 DE 19753363
(43) Veröffentlichungstag der Anmeldung: 06.12.2000
(73) Patentinhaber: Jenapharm GmbH & Co. KG, 07745 Jena (DE)
(72) Erfinder: SCHWARZ, Sigfrid, D-07743 Jena (DE); ELGER, Walter, D-14195 Berlin (DE); KAUFMANN, Günter, D-07743 Jena (DE); RÖMER, Wolfgang, D-07749 Jena (DE)
(74) Vertreter: Leybach, Holger Hugo
(86) Internationale Anmeldenummer: DE9803516
(87) Internationale Veröffentlichungsnummer: WO9928337

(56) Entgegenhaltungen:
- DE-A- 4 239 945
- DE-A- 4 239 946
- DE-A- 4 429 397
- DE-A- 4 429 398
- US-A- 3 766 224
- ZEELEN F J ET AL: "Structure-activity relationships of steroid estrogens" CYTOTOXIC ESTROGENS HORM. RECEPT. TUMORS, [PROC. WORKSHOP] (44VMAJ);1980; PP.39-48, XP002101004 Organon;Sci. Dev. Group; Oss; 5340 BH; Neth.

## Beschreibung

Es werden 15-Methyl-cyclopropanosteroide der allgemeinen Formel I, 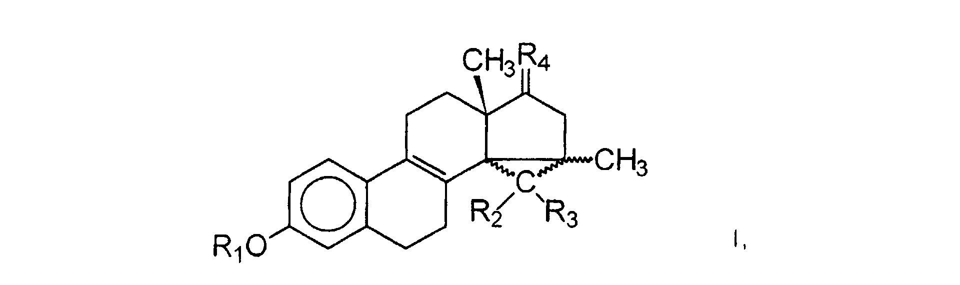 in der R₁ ein Wasserstoffatom, eine Alkylgruppe, eine Acylgruppe oder eine Sulfamoylgruppe bedeutet. R₂ und R₃ unabhängig voneinander ein Wasserstoffatom oder ein Halogenatom bedeuten, R₄ eine Oxogruppe oder ein Wasserstoffatom und eine Hydroxygruppe darstellt,und die 14,15-Methylengruppe entweder α- oder β-Konfiguration aufweist,
wobei die 15-Methylgruppe β-ständig ist, wenn die 14,15-Methylengruppe α- angeordnet ist und umgekehrt, beschrieben.

Bevorzugte Verbindungen sind dabei:
15β-Methyl-14α,15α-methylen-estra-1,3,5(10),8-tetraen-3,17α- diol
17α-Hydroxy-15β-methyl-14α,15α-methylenestra-1,3,5(10),8-tetraen-3-yl-sulfamat
17-Oxo-15β-methyl-14α,15α-methylenestra-1,3,5(10),8-tetraen-3-ylsulfamat
3-Methoxy-15α-methyl-14β,15β-methylenestra-1,3,5(10),8-tetraen-3,17β-ol
15β-Methyl-14α,15α-difluormethylenestra-1,3,5(10),8-tetraen-3,17α-diol
17α-Hydroxy-15β-methyl-14α,15α-difluormethylenestra-1,3,5(10),8-tetraen-3-yl-N,N-dimethylsulfamat
15α-Methyl-17-oxo-14β,15β-difluourmethylen-estra-1,3,5(10), 8-tetraen-3-yl-n-pentanoat
15-Methyl-estra-1,3,5(10),8,15-pentaen-3,17α-diol

Weiterhin werden 15-Methyl-pentaensteroide der allgemeinen Formel II 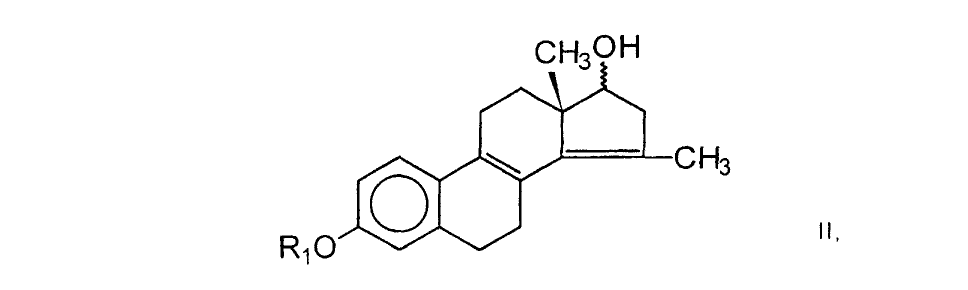 in der R₁ ein Wasserstoffatom bedeutet und die 17-Hydroxygruppe
α- oder β-ständig angeordnet ist, beschrieben,
die als Ausgangsverbindungen zur Herstellung der15-Methylcyclopropanosteroide der allgemeinen Formel I, 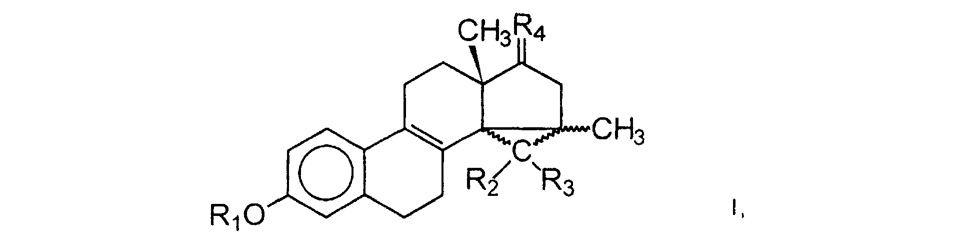 in der R₁ ein Wasserstoffatom, eine Alkylgruppe, eine Acylgruppe oder eine Sulfamoylgruppe bedeutet, R₂ und R₃ unabhängig voneinander ein Wasserstoffatom oder ein Halogenatom bedeuten, R₄ eine Oxogruppe oder ein Wasserstoffatom und eine Hydroxygruppe darstellt, und die 14,15-Methylengruppe entweder α- oder β-Konfiguration aufweist,
wobei die 15-Methylgruppe β-ständig ist, wenn die 14,15-Methylengruppe α- orientiert angeordnet ist und umgekehrt, geeignet sind.

Es wird auch ein Herstellungsverfahren der erfindungsgemäßen Verbindungen I und II beschrieben.

Aufgabe der Erfindung ist das Auffinden neuer Substanzen mit estrogener Wirksamkeit

Weiterhin ist Aufgabe der Erfindung, ein effektives und umweltschonendes-Verfahren zur Herstellung der erfindungsgemäßen Verbindungen zu entwickeln.

Die erfindungsgemäßen Verbindungen der allgemeinen Formel I sind aus neuen 15-Methyl-pentaensteroiden der allgemeinen Formel II, 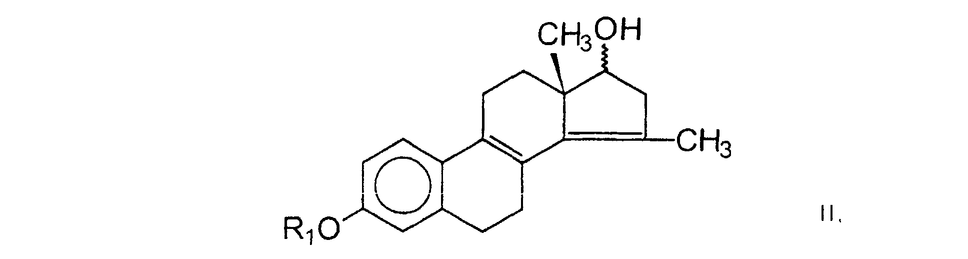 in der R₁ eine Alkylgruppe bedeutet und die 17-Hydroxygruppe α- oder β-ständig angeordnet ist, zugänglich,
indem diese Verbindungen in an sich bekannter Weise einer Cyclopropanierung der 14,15-Doppelbindung unterworfen werden, wobei 15-Methyl-cyclopropanosteroide der allgemeinen Formel I, in der R, eine Alkylgruppe, R₂ und R₃ unabhängig voneinander ein Wasserstoffatom oder ein Halogenatom bedeuten, R₄ ein Wasserstoffatom und eine Hydroxygruppe darstellt und die 14,15-Methylengruppe entweder α- oder β-Konfiguration aufweist, wobei die 15-Methylgruppe β-standig ist, wenn die 14,15-Methylengruppe α-angeordnet ist und umgekehrt, entstehen und mit diesen Verbindungen, in geeigneter Reihenfolge eine Spaltung des 3-Alkylethers mit nachfolgender Veresterung der 3-Hydroxygruppe mit Hilfe einer aktivierten Carbonsäure oder eines Sulfamoylchlorids und gegebenenfalls eine Oxidation der 17-Hydroxygruppe durchgeführt wird.

Beispielsweise wird durch Umsetzung des 15-Methyl-pentaensteroids der allgemeinen Formel II, in der R₁ eine Methylgruppe darstellt und die 17-Hydroxygruppe α-ständig ist mit Diiodmethan und Zink unter Ultraschalleinwirkung (modifizierte Simmons-Smith Reaktion) die erfindungsgemäße Verbindung der allgemeinen Formel I hergestellt, in der R₁ eine Methylgruppe bedeutet, R₂ und R₃ jeweils ein Wasserstoffatom darstellen, R₄ ein β-Wasserstoffatom und eine α-ständige Hydroxylgruppe bedeutet, die 14,15-Methylengruppe α-ständig und die 15-Methylgruppe β- angeordnet ist.
Etherspaltung, beispielsweise mit Diisobutylaluminiumhydrid in Toluen oder mit Natrium-ethylmercaptid in Dimethylsulfoxid liefert das erfindungsgemäße 15-Methyl-cyclopropanosteroid der allgemeinen Formel I, in der R₁, R₂ und R₃ jeweils ein Wasserstoffatom bedeuten, R₄ ein β-Wasserstoffatom und eine α-ständige Hydroxygruppe darstellt, die 14,15-Methylengruppe α-ständig und die 15-Methylgruppe β- angeordnet ist.
Geht man von dem 15-Methyl-pentaensteroid der allgemeinen Formel II, in der R₁ eine Methylgruppe darstellt und die 17-Hydroxygruppe β-ständig ist, aus und in analoger Weise vor, entsteht das 15-Methylcycolpropanosteroid der allgemeinen Formel I, in der R₁, R₂ und R₃ jeweils ein Wasserstoffatom bedeuten, R₄ ein α-Wasserstoffatom und eine β-ständige Hydroxylgruppe bedeutet, die 14,15-Methylengruppe β-ständig und die 15-Methylgruppe α- angeordnet ist.
Reagiert hingegen das 15-Methyl-pentaensteroid der allgemeinen Formel II, in der R₁ eine Methylgruppe darstellt und die 17-Hydroxygruppe α-ständig ist, mit Natrium-chlordifluoracetat in einem geeigneten hochsiedenden Lösungsmittel, dann entsteht das erfindungsgemäße 15-Methyl-cyclopropanosteroid der allgemeinen Formel I, in der R₁ eine Methylgruppe bedeutet, R₂ und R₃ jeweils ein Fluoratom darstellen, R₄ eine α-ständige Hydroxylgruppe und ein β-Wasserstoffatom bedeutet, die Difluormethylenbrücke α-ständig ist und die 15-Methylgruppe β-Konfiguration aufweist.
Oxidation dieser Verbindung mit Dimethylsulfoxid / Pyridin-Schwefeltrioxid-Komplex ergibt das erfindungsgemäße 15-Methyl-cyclopropanosteroid der allgemeinen Formel I, in der R₁ eine Methylgruppe darstellt, R₂ und R₃ jeweils ein Fluoratom bedeuten und R₄ eine Oxogruppe darstellt.

Verbindungen der allgemeinen Formel II sind ihrerseits aus Cyclopropano-tetraensteroiden der allgemeinen Formel III 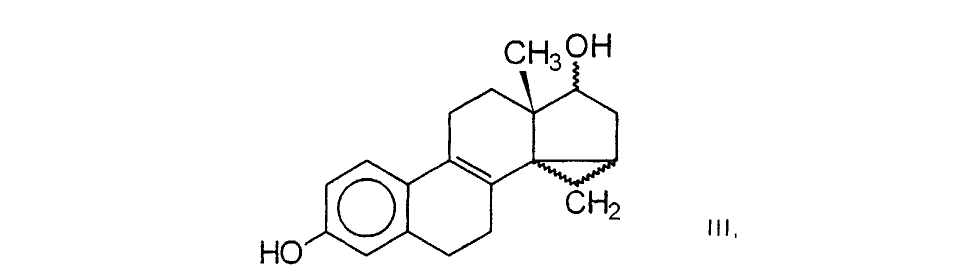 zugänglich,
in der die 17-Hydroxygruppe α- oder β-ständig ist und die 14,15-Methylenbrücke α- oder β-orientiert ist,
indem diese Cyclopropano-tetraensteroide zunächst einer thermischen Isomerisierung unterworfen werden, die zu 15-Methyl-pentaensteroiden der allgemeinen Formel II führt, in der R₁ ein Wasserstoffatom darstellt und die 17-Hydroxygruppe α- oder β-ständig ist
und dann die 3-Hydroxygruppe dieser Verbindungen in an sich bekannter Weise alkyliert wird.

Die thermische Isomerisierung des Cyclopropano-teraensteroids der allgemeinen Formel III wird in einem Temperaturbereich von +180 °C bis +220 °C in der Schmelze durchgeführt.
Diese Verfahrensweise vermeidet die Verwendung von organischen Lösungsmitteln, wodurch der Prozess nicht nur wirtschaftlich, sondern auch ökologisch günstig gestaltet werden kann.

Cyclopropano-tetraensteroids der allgemeinen Formel III, in der die 14,15-Methylenbrücke und die 17-Hydroxygruppe α-ständig sind, und mittels der erfindungsgemäßen thermischen Isomerisierung umgewandelt werden, sind in der DE 42 39 945 A1 aufgezeigt.
1-Stündiges Schmelzen dieser Verbindung bei+200 °C ergibt das 15-Methyl-pentaensteroid der allgemeinen Formel II, in der R₁ ein Wasserstoffatom darstellt und die 17-Hydroxygruppe α-ständig ist, in praktisch quantitativer Ausbeute.

Verbindungen der allgemeinen Formel I weisen estrogene Wirkungen auf, wodurch sie vorzugsweise für den Einsatz in der Hormonreplacement - Therapie bei der Frau geeignet sind. Die erfindungsgemäßen Verbindungen können oral oder parenteral appliziert werden.

Die erfindungsgemäßen Verbindungen und ihre Herstellung werden nachfolgend beispielhaft, aber nicht einschränkend, beschrieben.

### Beispiel 1

### Thermische Isomerisierung von 14α,15α-Methylenestra-1,3,5(10),8-tetraen-3,17α-diol zu 15-Methylestra-1,3,5(10),8,14-pentaen-3,17α-diol

14α,15α-Methylenestra-1,3,5(10),8-tetraen-3,17α-diol (10 g), das sich in einem Rundkolben befindet, wird in einem Metallbad auf +180 °C erwärmt. Hierbei bildet sich eine Schmelze, die sich nach 1 Stunde wieder zu rekristallisieren beginnt. Nach 5 Stunden läßt man abkühlen, rührt den kristallinen Rückstand in Aceton (40 ml) auf, saugt die Suspension über eine Glasfriite ab, wäscht den Filterrückstand mit kaltem Aceton und trocknet diesen, wobei man 15-Methyl-estra-1,3,5(10),8 14-pentaen-3,17α-diol erhält.
¹H-NMR (ppm *vs.* TMS; DMSO-d₆): 0,816 (H₁₈), 1,915 (15-CH₃), 3,644 (H_{17β}), 4,41(17α-OH), 6,55, 6,57 (H₂+H₄), 7,131 (H₁), 9,294 (3-OH)

### Beispiel 2

### Veretherung von 15-Methylestra-1,3,5(10),8,14-pentaen-3,17α-diol zu 3-Methoxy-15-methylestra-1,3,5(10),8,14-pentaen-17α-ol

15-Methylestra-1,3,5(10),8,14-pentaen-3,17α-diol (5,46 g), Kaliumcarbonat (5,30 g), Dimethylsulfat (3,61 ml) und Aceton (217 ml) werden unter Rühren 24 Stunden lang am Rückfluß erhitzt. Danach destilliert man das Aceton im Vakuum weitgehend ab und versetzt den Rückstand mit Wasser (300 ml) Dieses Gemisch extrahiert man mit Ethylacetat (400 ml). Der Extrakt wirde 3 mal mit Wasser (je 100 ml) gewaschen, über wasserfreiem Natriumsulfat getrocknet und am Rotationsverdampfer eingeengt. Den Rückstand trocknet man 1 Stunde bei Raumtemperatur an der Ölpumpe. Danach setzt man das erhaltene 3-Methoxy-15-methylestra-1,3,5(10),8,14-pentaen-17α-ol ohne weitere Reinigung in die nächste Stufe ein.
¹H-NMR (ppm *vs.* TMS; DMSO-d₆): 0,823 (H₁₈), 1,927 (15-CH₃), 3,738 (3-OCH₃), 3,650 (H_{17β}), 4,4 (17α-OH), 6,742 (H₂+H₄), 7,242 (H₁).

### Beispiel 3

### Cyclopropanierung von 3-Methoxy-15-methylestra-1,3,5(10),8,14-pentaen-17α-ol zu 15β-Methyl-3-methoxy-14α,15α-methylenestra-1,3,5(10),8-tetraen-17α-ol

Zinkstaub (27 g), Tetrahydrofuran (120 ml) und eine Spatelspitze lod werden im Ultraschallbad unter Argonschutz gerührt. Nach 5 Minuten gibt man 15β-Methyl-3-methoxy-14α,15α-methylenestra-1,3,5(10),8-tetraen-17α-ol (6.9 g) zu und erwärmt das Gemisch auf +70 °C. Danach läßt man Diiodmethan (7.2 ml) langsam zutropfen. Nach 5,5 stündiger Reaktion kühlt man das Gemisch auf +10 °C ab. Durch Zugabe von Ethylacetat (200 ml) und wässriger Ammoniumchloridlosung (900 ml 20 %) wird der Ansatz zersetzt. Man filtriert, trennt die Phasen des Filtrats, wäscht die organische Phase mit wässriger Ammoniumchloridlösung (4 mal mit je 400 ml, 20 %), mit wäßriger Natriumthiosulfatlösung (500 ml, 5 %) und mit Wasser (400 ml), trocknet die organische Phase über wasserfreiem Natriumsulfat und engte im Vakuum zur Trockene ein. Der Rückstand wird an Kieselgel chromatographiert (Cyclohexan:Ethylacetat 7:2), wobei man 15β-Methyl-3-methoxy-14α,15α-methylenestra-1,3,5(10),8-tetraen-17α-ol erhält.
¹H-NMR (ppm *vs.* TMS; CDCl₃): 0,928 (H₁₈), 1,465 (15-CH₃), 3,784 (H_{17β}),3,797 (3-OCH3), 6,679 (H₄), 6,71 (H₂), 7,147 (H₁).

### Beispiel 4

### Etherspaltung von 15α-Methyl-3-methoxy-14β,15β-methylenestra-1,3,5(10),8-tetraen-17α-ol zu 15α-Methyl-14β,15β-methylenestra-1,3,5(10),8-tetra- en-3,17α-diol

Zu einer auf 0 °C abgekühlten Mischung aus 15α-Methyl-3-methoxy-14β,15β-methylenestra-1.3.5(10),8-tetraen-17α-ol (0,54 g) und Toluen (14 ml) gibt man unter Argonschutz und Feuchtigkeitsausschluß tropfenweise Diisobutylaluminiumhydrid (2,7 ml, gelöst in 5,4 ml Toluen) Diese Lösung erwärmt man 3 Stunden zum Sieden am Rückfluß Danach kühlt man auf 0 °C ab. tropft vorsichtig Ethanol (8 ml, 95 %) und danach ein Gemisch aus konzentrierter Chlorwasserstoffsaure und Wasser (1:1, 14 ml) zu. Hierbei steigt die Temperatur des Reaktionsgemisches auf 10 °C bis 15 °C an. Man extrahiert mit Ethylacetat (30 ml), wäscht die organische Phase mit gesättigter wäßriger Natriumhydrogencarbonatlösung,(30 ml) und 2 mal mit gesättigter wäßriger Natriumchloridlösung (je 30 ml) Anschließend trocknet man den Extrakt über wasserfreiem Magnesiumsulfat und engt im Vakuumrotationsverdampfer ein. Der Rückstand wird an Kieselgel chromatographiert (Cyclohexan:Ethylacetat 3:2). Die Fraktionen, die die Titelverbindung enthalten, werden vereinigt und im Vakuum zur Trokene eingeengt Das Produkt löst man in Aceton und nach Zugabe von n-Hexan kristallisierte 15α-Methyl-14β,15β-methylenestra-1,3,5(10),8-tetra- en-3,17α-diol.
¹H-NMR (ppm *vs.* TMS; CDCl₃): 0,675 (14,15-CH₂), 0,886 (14,15-CH₂), 0,951 (H₁₈), 1,1913 (15α-CH₃), 3,540 (H_{17β}), 6,639 (H₄), 6,669 (H₂), 7,215 (H₁).

### Beispiel 5

### Veresterung von 15β-methyl-14α,15α-methylenestra-1,3,5(10),8-tetraen-3,17α-diol zu 17α-Hydroxy-15β-methyl-14α,15α-methylenestra-1,3,5(10),8-tetraen-3-yl sulfamat

Zu einer Lösung von 15β-methyl-14α,15α-methylenestra-1,3,5(10),8-tetraen-3,17α-diol (1,12 g) in Dichlormethan (48 ml) und 2,6-Ditert.butyl-4-methylpyridin 1,84 g) gibt man unter Rühren bei Raumtemperatur Sulfamoylchlorid (2,07 g). Man läßt 1 Stunde bei Raumtemperatur reagieren, wäscht die Lösung dann mit Wasser neutral, trocknet über wasserfreiem Natriumsulfat und engt im Vakuumrotationsverdampfer ein.Chromatographie an Kieselgel
(Toluen:Chloroform:Methanol 80:15:5) ergibt 17α-Hydroxy-15β-methyl-14α,15α-methylenestra-1,3,5(10),8-tetraen-3-yl sulfamat.
¹H-NMR (ppm *vs.* TMS; DMSO-d₆): 0,415 (14,15-CH₂), 0,837 (H₁₈), 1,412 (15-CH₃), 3,583 (H_{17β}), 4,530 (17α-OH), 7,009 (H₄), 7,049 (H₂), 7,208 (H₁), 7,926 (NH₂).

## Patentansprüche

1. 15-Methyl-cyclopropanosteroide der allgemeinen Formel I 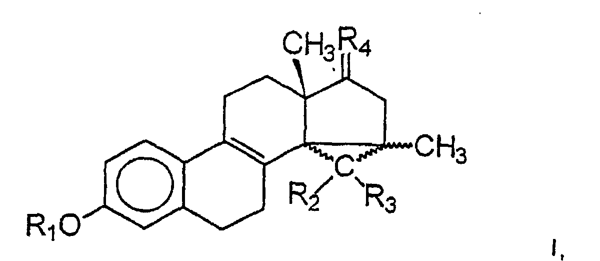 in der
R₁ ein Wasserstoffatom, eine Alkylgruppe, eine Acylgruppe oder eine Sulfamoylgruppe bedeutet,
R₂ und R₃ unabhängig voneinander ein Wasserstoffatom oder ein Halogenatom bedeuten,
R₄ eine Oxogruppe oder ein Wasserstoffatom und eine Hydroxygruppe darstellt
und die 14,15-Methylengruppe entweder α- oder β-Konfiguration aufweist,
wobei die 15-Methylgruppe β-ständig ist, wenn die 14,15-Methylengruppe α-angeordnet ist und umgekehrt.

2. 15β-Methyl-14α,15α-methylenestra-1,3,5(10),8-tetraen-3,17α-diol
15α-Methyl-14β,15β-methylenestra-1,3,5(10),8-tetraen-3,17β-diol
17α-Hydroxy-15β-methyl-14α,15α-methylenestra-1,3,5(10),8-tetraen-3-yl-sulfamat
17-Oxo-15β-methyl-14α,15α-methylenestra-1,3,5(10),8-tetraen-3-yl-sulfamat
3-Methoxy-15α-methyl-14β,15β-methylenestra-1,3,5(10),8-tetraen-3,17β-ol
15β-Methyl-14α,15α-difluormethylenestra-1,3,5(10),8-tetraen-3,17α-diol
17α-Hydroxy-15β-methyl-14α,15α-difluormethylenestra-1,3,5(10),8-tetraen-3-yl-N,N-dimethylsulfamat
15α-Methyl-17-oxo-14β,15β-difluourmethylen-estra-1,3,5(10),8-tetraen-3-yl-n-pentanoat

3. 15-Methyl-pentaensteroide der allgemeinen Formel II 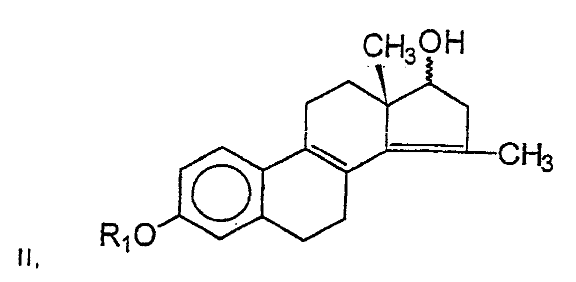 in der
R₁ eine Alkylgruppe bedeutet und die
17-Hydroxygruppe α- oder β-ständig ist,
als Ausgangsverbindungen zur Herstellung der Verbindungen gemäß Anspruch 1.

4. Verfahren zur Herstellung von 15-Methyl-cyclopropanosteroiden nach den Ansprüchen 1 und 2,
**dadurch gekennzeichnet,**
**daß** man Cyclopropano-tetraensteroide der allgemeinen Formel III 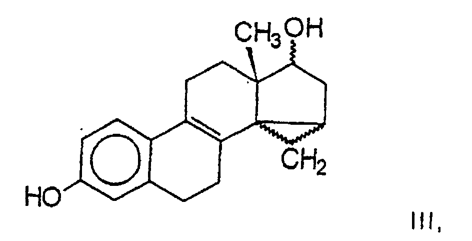 in der
die 17-Hydroxygruppe α- oder β-ständig ist und
die 14,15-Methylenbrücke α- oder β-orientiert ist, einer thermischen Isomerisierung unterwirft, wobei 15-Methyl-pentaensteroide der allgemeinen Formel II 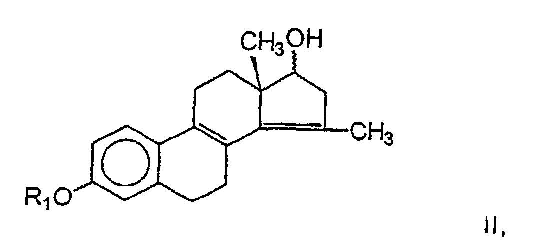 in der
R₁ ein Wasserstoffatom bedeutet und
die 17-Hydroxygruppe α- oder β-ständig angeordnet ist, entstehen,
diese in an sich bekannter Weise einer Alkylierung der 3-Hydroxygruppe und einer Cyclopropanierung der 14,15-Doppelbindung unterwirft und mit den hierbei entstehenden 15-Methyl-cyclopropanosteroiden der allgemeinen Formel I, 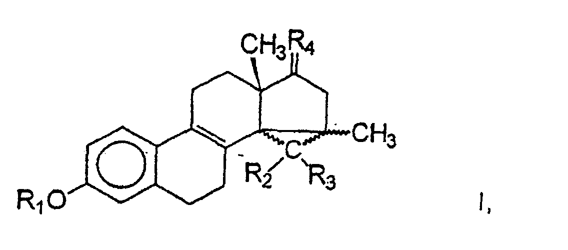 in der
R₁ eine Alkylgruppe bedeutet,
R₂ und R₃ unabhängig voneinander ein Wasserstoffatom oder ein Halogenatom bedeuten,
R₄ ein Wasserstoffatom und eine Hydroxygruppe darstellt und
die 14,15-Methylengruppe entweder α- oder β-Konfiguration aufweist,
wobei die 15-Methylgruppe β-ständig ist, wenn die 14,15-Methylengruppe α-angeordnet ist und'
umgekehrt,
in geeigneter Reihenfolge eine Spaltung des 3-Alkylethers und nachfolgend eine Veresterung der 3-Hydroxygruppe mit Hilfe einer aktivierten Carbonsäure oder eines Sulfamoylchlorids und gegebenenfalls eine Oxidation der 17-Hydroxygruppe vornimmt.

5. verfahren nach Anspruch 3, **dadurch gekennzeichnet, daß** die thermische Isomerisierung von Cyclopropano-tetraensteroiden der allgemeinen Formel III zu 15-Methyl-pentaensteroiden der allgemeinen Formel II bei +180 °C bis + 220 °C in der Schmelze durchgeführt wird.

6. Pharmazeutische Zusammensetzung, die mindestens eine Verbindung der allgemeinen Formel (I) nach -einem der Ansprüche 1 und 2, gegebenenfalls zusammen mit pharmazeutisch verträglichen Hilfs- und Trägerstoffen enthält.

7. Verwendung einer Verbindungen der allgemeinen Formel (I) gemäß einem der Ansprüche 1 und 2 zur Herstellung eines Arzneimittels zur Hormon-Replacement-Therapie (HRT) bei der Frau.

8. Verbindungen der allgemeinen Formel (I) nach einem der Ansprüche 1 und 2 zur Anwendung als therapeutische Wirkstoffe.

## Claims

1. 15-Methylcyclopropano steroids of the general formula I 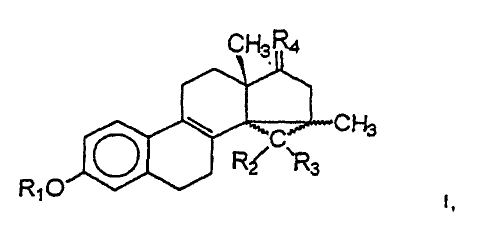 where
R₁ is a hydrogen atom, an alkyl group, an acyl group or a sulphamoyl group,
R₂ and R₃ are independently a hydrogen atom or a halogen atom,
R₄ is an oxo group or a hydrogen atom and a hydroxyl group
the 14,15-methylene group has either α- or β-configuration
and the 15-methyl group is in the β-position when the 14,15-methylene group is in the α-position and vice versa.

2. 15β-Methyl-14α,15α-methyleneestra-1,3,5(10),8-tetraene-3,17α-diol
15α-methyl-14β,15β-methyleneestra-1,3,5(10),8-tetraene-3,17β-diol
17α-hydroxy-15β-methyl-14α,15α-methyleneestra-1,3,5(10),8-tetraen-3-yl sulphamate
17-oxo-15β-methyl-14α,15α-methyleneestra-1,3,5(10),8-tetraen-3-yl sulphamate
3-methoxy-15α-methyl-14β,15β-methyleneestra-1,3,5(10),8-tetraen-3,17β-ol
15β-methyl-14α,15α-difluoromethyleneestra-1,3,5(10),8-tetraene-3,17α-diol
17α-hydroxy-15β-methyl-14α,15α-difluoromethyleneestra-1,3,5(10),8-tetraen-3-yl-N,N-dimethyl sulphamate
15α-methyl-17-oxo-14β,15β-difluoromethyleneestra-1,3,5(10),8-tetraen-3-yl n-pentanoate.

3. 15-Methylpentaene steroids of the general formula II 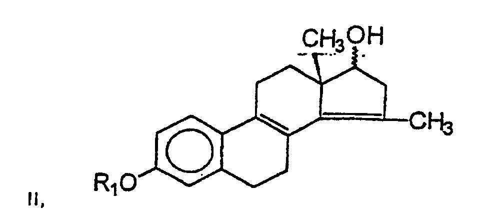 where R₁ is an alkyl group and
the 17-hydroxyl group is in the α- or β-position, used as starting compounds for preparing the compounds according to Claim 1.

4. Process for preparing 15-methylcyclopropano steroids according to Claims 1 and 2, **characterized in that** cyclopropanotetraene steroids of the general formula III 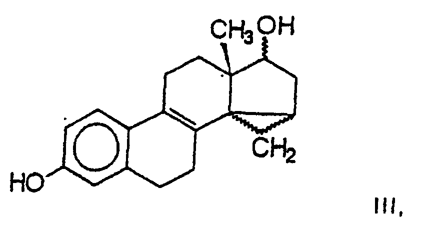 where
the 17-hydroxyl group is in the α- or β-position and
the 14,15-methylene bridge is α- or β-orientated, are subjected to a thermal isomerization, which gives 15-methylpentaene steroids of the general formula II 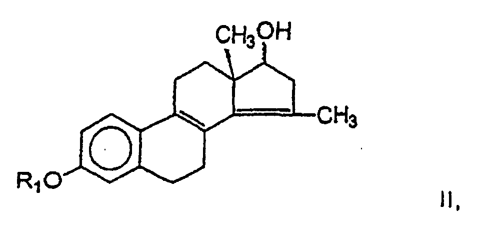 where
R₁ is a hydrogen atom and
the 17-hydroxyl group is in the α- or β-position, and these are then subjected to a conventional alkylation of the 3-hydroxyl group and a cyclopropanation of the 14,15-double bond to give 15-methylcyclopropano steroids of the general formula I 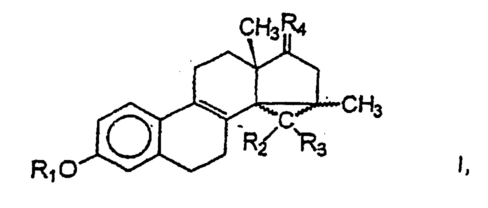 where
R₁ is an alkyl group
R₂ and R₃ are independently a hydrogen atom or a halogen atom
R₄ is a hydrogen atom and a hydroxyl group and
the 14,15-methylene group has either α- or β-configuration
and the 15-methyl group is in the β-position when the 14,15-methylene group is in the α-position and vice versa,
and are subjected in a suitable order to cleavage of the 3-alkyl ether and subsequent esterification of the 3-hydroxyl group with the aid of an activated carboxylic acid or a sulphamoyl chloride and optionally oxidation of the 17-hydroxyl group.

5. Process according to Claim 3, **characterized in that** the thermal isomerization of cyclopropanotetraene steroids of the general formula III to give 15-methylpentaene steroids of the general formula II is carried out at from +180°C to +220°C in the melt.

6. Pharmaceutical composition which comprises at least one compound of the general formula (I) according to Claim 1 or 2, optionally together with pharmaceutically acceptable adjuvants and supports.

7. Use of a compound of the general formula (I) according to Claim 1 or 2 for preparing a drug for hormone replacement therapy (HRT) in women.

8. Compounds of the general formula (I) according to Claim 1 or 2 for use as therapeutics.

## Revendications

1. 15-méthyl-cyclopropanostéroïdes de formule générale I 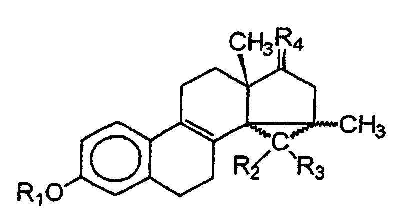 dans laquelle
R₁ représente un atome d'hydrogène, un groupe alkyle, un groupe acyle ou un groupe sulfamoyle,
R₂ et R₃ représentent, indépendamment l'un de l'autre, un atome d'hydrogène ou un atome d'halogène,
R⁴ représente un groupe oxo ou un atome d'hydrogène et un groupe hydroxy,
et le groupe 14,15-méthylène présente une configuration soit α soit β,
le groupe 15-méthyle étant en position β si le groupe 14,15-méthylène est en position α et inversement.

2. 15β-méthyl-14α,15α-méthylène-estra-1,3,5(10),8-tétraène-3,17α-diol
15α-méthyl-14β,15β-méthylène-estra-1,3,5(10),8-tétraène-3,17β-diol
17α-hydroxy-15β-méthyl-14α,15α-méthylène-estra-1,3,5(10),8-tétraén-3-yl-sulfamate
17-oxo-15β-méthyl-14α,15α-méthylène-estra-1,3,5(10),8-tétraén-3-yl-sulfamate
3-méthoxy-15α-méthyl-14β,15β-méthylène-estra-1,3,5(10),8-tétraén-3,17β-ol
15β-méthyl-14α,15α-difluorométhylène-estra-1,3,5(10),8-tétraène-3,17α-diol
17α-hydroxy-15β-méthyl-14α,15α-difluorométhylène-estra-1,3,5(10),8-tétraén-3-yl-N,N-diméthylsulfamate
15α-méthyl-17-oxo-14β,15β-difluorométhylène-estra-1,3,5(10),8-tétraén-3-yl-n-pentanoate.

3. 15-méthyl-pentaènestéroïdes de formule générale II 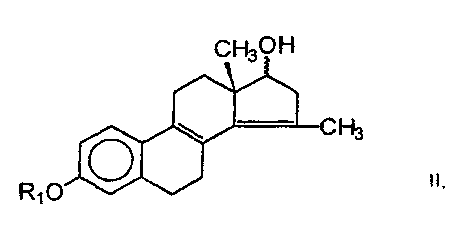 dans laquelle
R₁ représente un groupe alkyle et
le groupe 17-hydroxy est en position α ou β,
en tant que composés de départ pour la préparation des composés selon la revendication 1.

4. Procédé de préparation de 15-méthyl-cyclopropanostéroïdes selon les revendications 1 et 2, **caractérisé en ce que** l'on soumet des cyclopropanotétraènestéroïdes de formule générale III 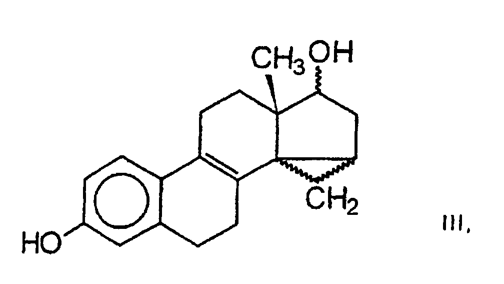 dans laquelle
le groupe 17-hydroxy est en position α ou β et
le pont 14,15-méthylène est à orientation α ou β, à une isomérisation thermique, en formant des 15-méthyl-pentaènestéroïdes de formule générale II 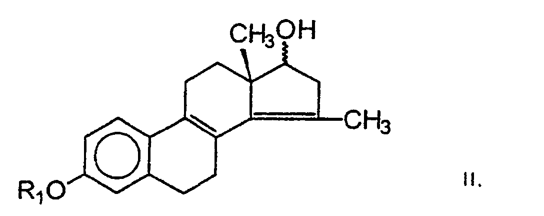 dans laquelle
R₁ représente un atome d'hydrogène et
le groupe 17-hydroxy est en position α ou β, on soumet ces derniers, de façon connue en soi, à une alkylation du groupe 3-hydroxy et une cyclopropanation de la double liaison en 14,15 et on procède, avec les 15-méthyl-cyclopropanostéroïdes de formule générale I ainsi formés, 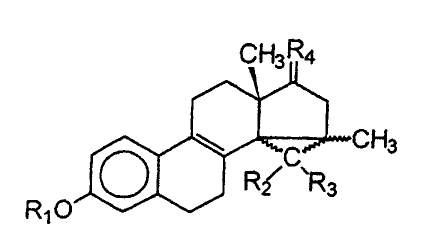 dans laquelle
R₁ représente un groupe alkyle,
R₂ et R₃ représentent, indépendamment l'un de l'autre, un atome d'hydrogène ou un atome d'halogène,
R₄ représente un atome d'hydrogène et un groupe hydroxy, et
le groupe 14,15-méthylène présente une configuration soit α soit β,
le groupe 15-méthyle étant en position β si le groupe 14,15-méthylène est en position α et inversement,
dans un ordre approprié, à un clivage de l'éther 3-alkylique et ensuite à une estérification du groupe 3-hydroxy à l'aide d'un acide carboxylique activé ou d'un chlorure de sulfamoyle et éventuellement à une oxydation du groupe 17-hydroxy.

5. Procédé selon la revendication 3, **caractérisé en ce que** l'isomérisation thermique de cyclopropanotétraènestéroïdes de formule générale III en 15-méthyl-pentaènestéroïdes de formule générale II est réalisée à l'état fondu entre +180°C et +220°C.

6. Composition pharmaceutique comprenant au moins un composé de formule générale (I) selon l'une quelconque des revendications 1 et 2, éventuellement conjointement avec des auxiliaires et des supports pharmaceutiquement acceptables.

7. Utilisation d'un composé de formule générale (I) selon l'une quelconque des revendications 1 et 2, pour la préparation d'un médicament destiné à l'hormonothérapie substitutive (HTS) chez la femme.

8. Composés de formule générale (I) selon l'une quelconque des revendications 1 et 2, destinés à être utilisés en tant qu'ingrédients actifs thérapeutiques.
